Europäisches Patentamt

European Patent Office

Office européen des brevets

(11) Veröffentlichungsnummer : **0 495 804 B1**

(12) **EUROPÄISCHE PATENTSCHRIFT**

(45) Veröffentlichungstag der Patentschrift :
18.05.94 Patentblatt 94/20

(21) Anmeldenummer : 90914503.9

(22) Anmeldetag : 06.10.90

(86) Internationale Anmeldenummer :
PCT/DE90/00763

(87) Internationale Veröffentlichungsnummer :
WO 91/05994 02.05.91 Gazette 91/10

(51) Int. Cl.⁵ : **G01N 15/06**

(54) **VERFAHREN UND MESSANORDNUNG ZUR BESTIMMUNG DES RUSSGEHALTES IN ABGASEN.**

(30) Priorität : 21.10.89 DE 3935149

(43) Veröffentlichungstag der Anmeldung :
29.07.92 Patentblatt 92/31

(45) Bekanntmachung des Hinweises auf die
Patenterteilung :
18.05.94 Patentblatt 94/20

(84) Benannte Vertragsstaaten :
CH DE ES FR GB IT LI SE

(56) Entgegenhaltungen :
EP-A- 0 071 474

(56) Entgegenhaltungen :
CH-A- 667 534
DE-C- 3 305 867
US-A- 4 633 706
US-A- 4 747 297

(73) Patentinhaber : **ROBERT BOSCH GMBH**
**Postfach 30 02 20**
**D-70442 Stuttgart (DE)**

(72) Erfinder : **FRIESE, Karl-Hermann**
**Strohgaeustrasse 13**
**D-7250 Leonberg (DE)**
Erfinder : **HOETZEL, Gerhard**
**Mahlestrasse 62a**
**D-7000 Stuttgart 50 (DE)**

**Beschreibung**

Stand der Technik

Die Erfindung geht aus von einem Verfahren zur Bestimmung des Rußgehaltes in Abgasen, insbesondere den Abgasen von Dieselmotoren.

Es ist allgemein bekannt, daß sich in Abgasen von zur Verbrennung gebrachten Brennstoffen, z. B. in Abgasen von Dieselmotoren, Rußpartikel befinden, die die Umwelt in hohem Maße belasten. Es sind daher starke Bestrebungen im Gange, den Ausstoß von Rußpartikeln zu vermindern.

Zur Bestimmung des Rußgehaltes von Abgasen sind sehr verschiedene Verfahren bekannt geworden.

Eine Standardmeßmethode zur quantitativen Bestimmung der mit einem Abgas ausgestoßenen Rußpartikel besteht darin, daß man Rußpartikel über einen bestimmten Zeitraum mit Hilfe eines Filters, das im Abgaskanal, z. B. im Auspuffrohr eines Kraftwagens angeordnet ist, abfiltriert, daß man die abfiltrierten Rußpartikel in geeichte Gefäße bringt, bis zur Erreichung eines konstanten Gewichtes trocknet und kühlt und dann auswiegt. Dieses Verfahren ist weit verbreitet und liegt vielfach gesetzlichen Bestimmungen zugrunde. Es eignet sich jedoch nicht zur Durchführung von kontinuierlichen Messungen oder gar zur Steuerung des Luft/Kraftstoffverhältnisses von Dieselmotoren.

Über neuere Meßverfahren zur Ermittlung dieselmotorischer Emission wird von H. Holtei in den VDI-Berichten Band 559, 1985, Seiten 319-334 berichtet.

Ein Rußprüfgerät für Abgase von Dieselfahrzeugen, bei dem ein bestimmtes Abgasvolumen mittels einer Pumpe mit einem elektronischen Antrieb durch ein Filterpapier gesaugt wird und aus dem Schwärzungsgrad des Filterpapiers an der Stelle, an der es von den Abgasen durchströmt wird, durch Vergleich mit einer Schwärzungstabelle die sog. Rußzahl oder ein anderer für den Rußgehalt des Abgases charakteristischer Wert ermittelt wird, ist aus der DE-OS 36 15 111 bekannt.

Aus der US-PS 4 633 706 ist ferner ein Verfahren zur Bestimmung des Rußgehaltes von Verbrennungsmotoren, insbesondere Dieselmotoren bekannt, das auf der Ermittlung des Druckabfalles eines im Abgaskanal angeordneten Filters beruht, mit dem die Rußpartikel des Abgases abgefangen werden.

Aus der DE-PS 34 14 542 ist weiterhin eine Vorrichtung zur Bestimmung des Rußgehaltes von Abgasen mit zwei spannungsbeaufschlagten Elektroden bekannt, mit der die Leitfähigkeit der Rußpartikel im Abgas kontinuierlich erfaßt werden kann. Zur Bestimmung des Anteils der Rußpartikel im Abgas wird dazu der Strom bzw. die Stromänderung, die durch eine Änderung der Leitfähigkeit zwischen den Elektroden hervorgerufen wird, gemessen.

Ein Detektor zur Bestimmung des Rußgehaltes von Abgasen, bei der der Rußgehalt mittels zweier Elektroden und eines die Rußpartikel abfangenden hitzewiderstandsfähigen Gliedes über durch die abgeschiedenen Rußpartikel herbeigeführte Widerstandsveränderungen ermittelt wird, ist ferner aus der US-PS 4 656 832 bekannt.

Aus der CH-PS 667 534 ist schließlich bereits ein Verfahren zur Messung des Partikelgehalts in Abgasen bekannt, bei dem mittels eines im Abgasstrom angeordneten Sensors ein darauf sich bildender partikelniederschlag erfaßt wird. Kennzeichnend für das bekannte Verfahren ist, daß als Sensor zwei elektrische Heizflächen vorgesehen werden, die in jeweils einem parallelen Brückenzweig einer Wheatstonschen Brückenschaltung angeordnet werden, daß eine Heizfläche ständig so vorgeheizt wird, daß der Partikelniederschlag auf ihrer Oberfläche minimal ist, daß während der Heizzeitdauer im Diagonalzweig der Wheatstonschen Brückenschaltung die Spannung und/oder die Spannungszunahme bzw. der Strom und/oder die Stromzunahme gemessen werden und daß aus dem Spannungs- bzw. Strommaximum und/oder der Steilheit des Spannungs- bzw. Stromanstiegs ein Warnsignal und/oder ein Meßwert für den Partikelgehalt abgeleitet wird.

Das bekannte Verfahren macht sich die Tatsache zunutze, daß die Entfernung des auf dem Sensor sich bildenden Partikelniederschlags, wie Ruß und dgl., ein endothermer Vorgang ist, also Wärme verbraucht. Die Heizenergie, die zum Aufheizen der Heizfläche zwecks Entfernung des Partikelniederschlags erforderlich ist, muß daher bei einer mit einem Partikelniederschlag belegten Heizfläche größer sein als bei einer sauberen Oberfläche der Heizfläche. Bei dem bekannten Verfahren wird daher die bisher zusätzlich erforderliche Reinigungsprozedur des Sensors zur Messung des Partikelgehalts selbst herangezogen.

Vorteile der Erfindung

Das erfindungsgemäße Verfahren mit den kennzeichnenden Merkmalen des Hauptanspruchs weist gegenüber den bisher bekanntgewordenen Verfahren zur Bestimmung des Rußgehaltes in Abgasen den besonderen Vorteil auf, daß der Rußgehalt indirekt über eine $O_2$-Differenzmessung erfolgt, so daß zur Durchführung des Verfahrens bewährte Sonden für die Bestimmung des Sauerstoffgehaltes von Abgasen eingesetzt werden können, die gegebenenfalls bei Durchführung des Verfahrens zur Bestimmung des Rußgehaltes in Abgasen von Dieselmotoren, gleichzeitig die Luft/Kraftstoffregelung übernehmen können.

Zur Durchführung des erfindungsgemäßen Verfahrens eignen sich somit bekannte Sonden, wie sie üblicherweise zur Messung der Sauerstoffkonzentration in Abgasen von Verbrennungskraftmaschinen eingesetzt werden, und die beispielsweise auch als sog. Lambda-Sonden bekannt geworden sind. Als besonders geeignet erwiesen haben sich dabei beheizte Zirconiumdioxid-Sonden, wie sie beispielsweise näher beschrieben werden in dem Sonderdruck aus der Zeitschrift Praxis der Naturwissenschaften Chemie", Heft 7/36,36. Jahrgang 1987 mit dem Titel "Beheizte Zirconiumdioxid-Sonde für stöchiometrische und magere Luft-Kraftstoffgemische", ferner in Bosch Technische Berichte 7 (1984), 5 sowie in dem SAE-Papier 840141, 1984. Anstatt dieser, die Form eines einseitig geschlossenen Rohres aufweisenden Sonden können jedoch auch sog. planare Grenzstrom-Magersonden, z. B. des aus den DE-OS 38 34 987, 38 11 713 und 38 33 541 bekannten Typs verwendet werden.

Das erfindungsgemäße Verfahren eignet sich ganz allgemein zur Bestimmung des Rußgehaltes in Abgasen von zur Verbrennung gebrachten fossilen Brennstoffen, insbesondere jedoch zur Bestimmung des Rußgehaltes in Abgasen von Dieselmotoren, wobei es zur Kontrolle der Abgasreinheit von Kraftfahrzeugen beispielsweise im Rahmen der technischen Überwachung von Kraftfahrzeugen mit einer zur Durchführung des Verfahrens geeigneten Vorrichtung (TÜV-Gerät) durchgeführt werden kann. Das erfindungsgemäße Verfahren läßt sich jedoch mit der zur Durchführung des Verfahrens geeigneten Meßanordnung auch in Kraftfahrzeugen durchführen.

Das Verfahren der Erfindung beruht somit auf der Erkenntnis, daß sich der Rußgehalt in Abgasen einfach und genau dadurch ermitteln läßt, daß man in einem von Abgas durchströmten Kanal mit mindestens einer hierin angeordneten Sonde für die Bestimmung des Sauerstoffgehaltes in Abgasen und einem beheizbaren Rußfilter

(a) die $O_2$-Konzentration des ungefilterten Abgases mißt;
(b) mit dem unbeheizten Rußfilter eine bestimmte Zeitspanne lang Rußpartikel abfiltriert;
(c) die abfiltrierten Rußpartikel durch Aufheizen des Rußfilters mit dem im Abgas vorhandenen Sauerstoff verbrennt;
(d) die durch Verbrennen der Rußpartikel verminderte $O_2$-Konzentration im Abgas mißt und
(e) aus dem verminderten $O_2$-Gehalt die Rußkonzentration des Abgases ermittelt,
wobei man die Verfahrensschritte (b) und (c) gegebenenfalls gleichzeitig durchführen kann.

Gemäß einer vorteilhaften Ausführungsform läßt sich das Verfahren in einem zweigeteilten Abgaskanal durchführen, wobei in beiden Teilen des Abgaskanals jeweils eine Sonde und vor einer Sonde ein Rußfilter angeordnet ist.

Die Erfindung ermöglicht somit auch die Durchführung eines kontinuierlichen Meßverfahrens bei Verwendung eines ständig beheizten Rußfilters und somit rußfreiem Gas in einer Kanalhälfte und rußhaltigem Abgas in der anderen Kanalhälfte.

Das erfindungsgemäße Verfahren läßt sich auch mit nur einer Sonde durchführen, und zwar dann, wenn die Sonde abwechselnd dem normalen Abgas oder dem Abgas mit der durch Verbrennung des abfiltrierten Rußes verminderten Sauerstoffkonzentration ausgesetzt werden kann, beispielsweise mittels eines rotierenden Systems.

Das beheizbare Rußfilter besteht in vorteilhafter Weise aus einem porösen hitzebeständigen keramischen Material, z. B. auf Zircondioxid- oder Aluminiumoxidbasis mit einem Gehalt an einem die Verbrennung von abgeschiedenem Ruß katalysierenden Material. Vorzugsweise besteht das Rußfilter aus einem zu einem Block verpreßten keramischen Material, der den Dimensionen des Abgaskanals angepaßt ist und sich leicht in diesen einsetzen läßt. Das die Verbrennung katalysierende Material kann beispielsweise aus Platin oder einer Platinlegierung bestehen.

Zeichnungen

Die Zeichnungen dienen der näheren Erläuterung der Erfindung. Dargestellt sind in

Fig. 1    die schematische Anordnung zweier Sonden S1 und S2 und eines Rußfilters in einem zweigeteilten Abgaskanal und in

Fig. 2    das zeitliche Meßsignal der Sonde S2.

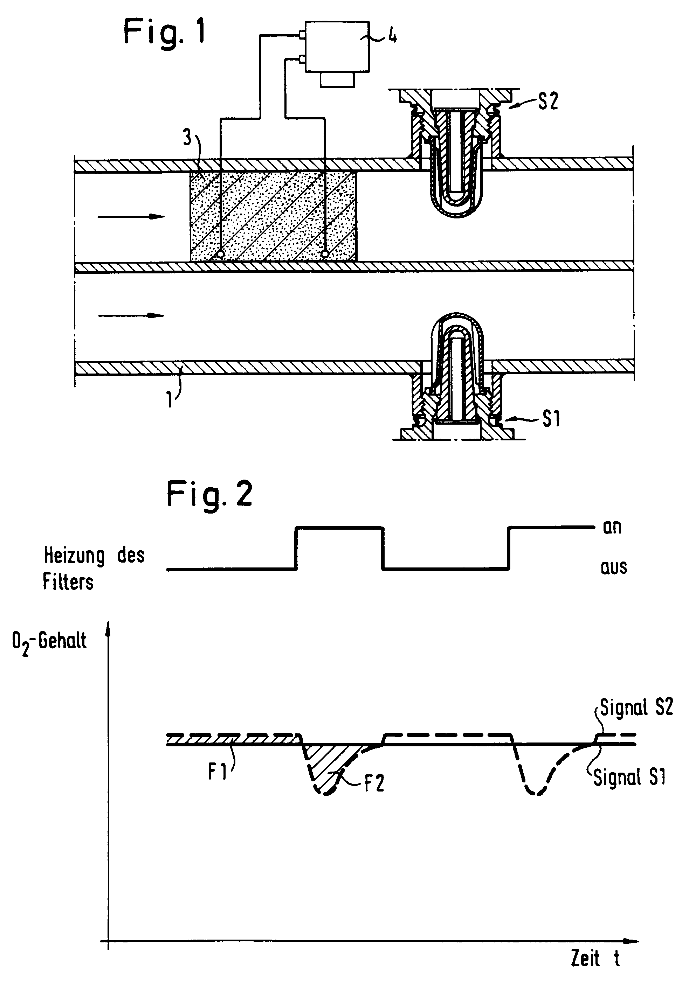

Wie in Fig. 1 schematisch dargestellt, besteht die zur Durchführung des erfindungsgemäßen Verfahrens verwendete Meßanordnung in ihren wesentlichen Teilen, vereinfacht dargestellt, aus den in einen zweigeteilten Abgaskanal 1 eingesetzten Sonden S1 und S2 sowie dem Rußfilter 3 mit der Aufheizvorrichtung 4. Bei den beiden Sonden S1 und S2 handelt es sich um bekannte Mager-Lambda-Sonden, d. h. beheizte Festelektrolyt-Sonden. Anstelle der die Form eines einseitig geschlossenen Rohres aufweisenden Festelektrolyt-Sonden, die nach dem Prinzip der galvanischen Sauerstoffkonzentrationskette arbeiten, können in der dargestellten Anordnung jedoch auch andere Sonden verwendet werden, insbesondere sog. planare Grenzstrom-Magersonden.

Das beheizbare, katalytisch aktives Material enthaltende Rußfilter 3 besteht aus einem in die eine Abgaskanalhälfte einsetzbaren, beheizbaren porösen Keramikblock, der aus dem den Abgaskanal in Pfeilrichtung durchströmenden Abgas die Rußpartikel abzufiltrieren vermag und der derart beheizbar ist, daß die in einer bestimmten Zeitspanne abfiltrierten Rußpartikel vollständig verbrannt werden können. Bei Beheizung des Filters werden somit die abfiltrierten Rußpartikel durch den im Abgas vorhandenen Sauerstoff zu $CO_2$ verbrannt. Infolgedessen liefert die Sonde S2 gegenüber der Sonde S1 ein entsprechend dem Rußgehalt des Abgases erniedrigtes Sauerstoffsignal. Somit gilt:

$$[O_2]_{S1} = [O_2]_{S2} = [O_2]$$

benötigt für die Rußverbrennung.

Bei vollständiger Verbrennung gilt:

$$[O_2]_{benötig} = f(Ruß)$$

Fig. 2 veranschaulicht die zeitlichen Meßsignale ($O_2$-Gehalt) der Sonden S1 und S2, wobei im Falle der Sonde S2 die Flächen F1 und F2 gleich groß sind und die Peakfläche F2 dem in der unbeheizten Zeit des Filters angereicherten Ruß proportional ist.

Gemäß einer vorteilhaften Ausgestaltung der Erfindung kann die Sonde S1 gleichzeitig zur Regelung des Luft/Kraftstoffverhältnisses herangezogen werden.

Im Falle der Fig. 1 befindet sich die Meßanordnung in einem zweigeteilten Abgaskanal. Die Meßanordnung läßt sich jedoch ebenso gut auch in einem mit dem Abgaskanal verbundenen Nebenkanal unterbringen, durch den nur ein Teil des gesamten Abgases geführt wird.

## Patentansprüche

1. Verfahren zur Bestimmung des Rußgehaltes in Abgasen, insbesondere den Abgasen von Dieselmotoren, dadurch gekennzeichnet, daß man in einem von dem Abgas durchströmten Kanal (1) mit mindestens einer hierin angeordneten Sonde ($S_1, S_2$) für die Bestimmung des Sauerstoffgehaltes in Abgasen und einem beheizbaren Rußfilter (3)

    (a) die $O_2$-Konzentration des ungefilterten Abgases mißt;
    (b) mit dem unbeheizten Rußfilter eine bestimmte Zeitspanne lang Rußpartikel abfiltriert;
    (c) die abfiltrierten Rußpartikel durch Aufheizen des Rußfilters mit dem im Abgas vorhandenen Sauerstoff verbrennt;
    (d) die durch Verbrennen der Rußpartikel verminderte $O_2$-Konzentration im Abgas mißt und
    (e) aus dem verminderten $O_2$-Gehalt die Rußkonzentration des Abgases ermittelt,
    wobei man die Verfahrensschritte (b) und (c) gegebenenfalls gleichzeitig durchführen kann.

2. Verfahren nach Anspruch 1, dadurch gekennzeichnet, daß man zur Bestimmung der $O_2$-Konzentration nach den Verfahrensstufen (a) und (d) die gleiche Sonde verwendet.

3. Verfahren nach Anspruch 1, dadurch gekennzeichnet, daß man das Abgas in einen zweigeteilten Abgaskanal (1) einführt und daß man in dem einen Kanalteil mittels einer ersten Sonder ($S_1$) die normale $O_2$-Abgaskonzentration und in dem anderen Kanalteil mittels einer zweiten Sonde ($S_2$) die durch Verbrennung abfiltrierter Rußpartikel verminderte $O_2$-Konzentration mißt.

4. Verfahren nach einem der Ansprüche 1 bis 3, dadurch gekennzeichnet, daß man als Rußfilter (3) einen porösen Keramikblock mit einem die Rußverbrennung katalysierenden Material verwendet.

5. Verfahren nach Anspruch 4, dadurch gekennzeichnet, daß man einen porösen Keramikblock mit Platin oder einer Platinlegierung als katalytisch aktivem Material verwendet.

6. Verfahren nach einem der Ansprüche 1 bis 5, dadurch gekennzeichnet, daß man als $O_2$-Meßsonden nach dem Prinzip der galvanischen Sauerstoffkonzentrationszelle arbeitende Sonden verwendet.

7. Verfahren nach Anspruch 6, dadurch gekennzeichnet, daß man Sonden auf Festelektrolytbasis verwendet.

8. Verfahren nach Anspruch 1, dadurch gekennzeichnet, daß man die Sonden gleichzeitig zur Steuerung des Luft/Kraftstoffverhältnisses verwendet.

**9.** Meßanordnung zur Durchführung des Verfahrens nach Ansprüchen 1 bis 8, bestehend aus einem zweigeteilten Abgaskanal (1) mit einer ersten und einer zweiten durch eine Kanalwand hindurch in den Kanal hineinragenden und gegenüber der Kanalwand isolierten Sonde ($S_1$,$S_2$) für die Bestimmung des $O_2$-Gehaltes von Abgasen, wobei vor der einen Sonde in Strömungsrichtung ein periodisch beheizbarer Rußfilter (3) aus porösem keramischem Material angeordnet ist.

**10.** Meßanordnung nach Anspruch 9, dadurch gekennzeichnet, daß eine der beiden Sonden ($S_1$,$S_2$) gleichzeitig zur Steuerung des Luft/Kraftstoffgemisches eines Dieselmotors ausgebildet ist.

## Claims

**1.** Method of determining the soot content in exhaust gases, in particular the exhaust gases of diesel engines, characterized in that, using at least one probe ($S_1$, $S_2$) which is disposed in a duct (1) having exhaust gas flowing through it and which is designed to determine the oxygen content in exhaust gases, and a heatable soot filter (3),

    (a) the $O_2$ concentration of the unfiltered exhaust gas is measured,

    (b) soot particles are filtered off for a certain time interval using the unheated soot filter,

    (c) the soot particles filtered off are combusted by heating the soot filter with the oxygen present in the exhaust gas,

    (d) the $O_2$ concentration in the exhaust gas, which concentration is reduced by combusting the soot particles, is measured, and

    (e) the soot concentration in the exhaust gas is determined from the reduced $O_2$ content,

it being optionally possible to carry out the method steps (b) and (c) simultaneously.

**2.** Method according to Claim 1, characterized in that the same probe is used to determine the $O_2$ concentration after the method steps (a) and (d).

**3.** Method according to Claim 1, characterized in that the exhaust gas is introduced into a two-part exhaust-gas duct (1) and in that the normal $O_2$ exhaust-gas concentration is measured in one duct part by means of a first probe and the $O_2$ concentration reduced by combusting the soot particles filtered off is measured in the other duct part by means of a second probe ($S_2$).

**4.** Method according to one of Claims 1 to 3, characterized in that a porous ceramic block containing a material catalyzing the soot combustion is used as soot filter (3).

**5.** Method according to Claim 4, characterized in that a porous ceramic block containing platinum or a platinum alloy is used as catalytically active material.

**6.** Method according to one of Claims 1 to 5, characterized in that probes which function in accordance with the principle of the galvanic oxygen-concentration cell are used as $O_2$-measuring probes.

**7.** Method according to Claim 6, characterized in that probes with a solid-electrolyte base are used.

**8.** Method according to Claim 1, characterized in that the probes are used simultaneously to control the air/fuel ratio.

**9.** Measuring device for carrying out the method according to Claims 1 to 8, comprising a two-part exhaust-gas duct (1) having a first and a second probe ($S_1$, $S_2$) projecting into the duct through a duct wall and insulated from the duct wall for determining the $O_2$ content of exhaust gases, a periodically heatable soot filter (3) made of porous ceramic material being disposed upstream of one probe in the flow direction.

**10.** Measuring device according to Claim 9, characterized in that one of the two probes ($S_1$, $S_2$) is designed simultaneously for controlling the air/fuel mixture of a diesel engine.

**Revendications**

1. Procédé pour déterminer la teneur en suie dans des gaz d'échappement, en particulier dans des gaz d'échappement de moteurs Diesel, procédé caractérisé en ce que, dans une canalisation parcourue par les gaz d'échappement (1) avec au moins une sonde (S1 et S2) disposée dedans servant à déterminer la teneur en oxygène dans les gaz d'échappement et avec un filtre à suie (3) qui peut être chauffé
   a) on mesure la concentration en oxygène des gaz d'échappement non filtrés,
   b) on filtre les particules de suie durant un intervalle de temps déterminé avec le filtre à suie, non chauffé,
   c) on brûle les particules de suie filtrées en chauffant le filtre à suie avec l'oxygène qui est disponible dans les gaz d'échappement,
   d) on mesure la concentration en oxygène dans les gaz d'échappement, qui se trouve réduite par la combustion des particules de suie,
   e) on détecte la concentration en suie des gaz d'échappement à partir de la teneur en oxygène, réduite, les séquences du procédé (b) et (c) pouvant être, le cas échéant exécutées en même temps.

2. Procédé selon la revendication 1, caractérisé en ce qu'on utilise, pour déterminer la concentration en oxygène selon les étapes (a) et (b) du procédé, la même sonde.

3. Procédé selon la revendication 1, caractérisé en ce qu'on introduit les gaz d'échappement dans une canalisation de gaz d'échappement en deux parties (1) et en ce qu'on mesure, dans l'une des parties de la canalisation au moyen d'une première sonde (S1), la concentration normale des gaz d'échappement en oxygène et dans l'autre partie de la canalisation au moyen d'une deuxième sonde (S2) la concentration en oxygène réduite par la combustion des particules de suie filtrées.

4. Procédé selon l'une des revendications 1 à 3, caractérisé en ce qu'on utilise comme filtre à suie (3) un bloc céramique poreux avec une matière qui catalyse la combustion de la suie.

5. Procédé selon la revendication 4, caractérisé en ce qu'on utilise un bloc céramique poreux avec du platine ou un alliage de platine comme matière catalytiquement active.

6. Procédé selon l'une des revendications 1 à 5, caractérisé en ce que l'on utilise comme sondes de mesure d'oxygène des sondes fonctionnant selon le principe des cellules galvaniques de concentration en oxygène.

7. Procédé selon la revendication 6, caractérisé en ce qu'on utilise des sondes à base d'électrolyte solide.

8. Procédé selon la revendication 1, caractérisé en ce qu'on utilise en même temps les sondes pour commander le rapport air/carburant.

9. Système de mesure pour la mise en oeuvre du procédé selon les revendications 1 à 8, consistant en une canalisation de gaz d'échappement en deux parties (1) avec une première et une deuxième sonde (S1, S2) pénétrant dans la canalisation à travers une de ses parois et isolées par rapport à la paroi du canal, servant à déterminer la teneur en oxygène des gaz d'échappement, système dans lequel est disposé avant l'une des sondes dans le sens de l'écoulement, un filtre à suie (3) qui peut être chauffé périodiquement et est en une matière céramique poreuse.

10. Dispositif de mesure selon la revendication 9, caractérisé en ce que l'une des deux sondes (S1, S2) est constituée en même temps pour commander le mélange air/carburant d'un moteur Diesel.

# Fig. 1

# Fig. 2

Heizung des
Filters

an

aus

$O_2$-Gehalt

Signal S2

F1

F2

Signal S1

Zeit t